# EUROPEAN PATENT SPECIFICATION

(11) **EP 4 565 059 B1**
(45) Date of publication and mention of the grant of the patent: **22.04.2026**
(21) Application number: 22761191.0
(22) Date of filing: 05.08.2022
(51) Int. Cl.: A01K 67/362, A01G 9/029, A01G 9/04, A01G 31/06, A01G 31/00, A01G 31/02

(54) **STACKABLE GROW BOX AND MODULAR GROW BOX SYSTEM**
STAPELBARE WACHSTUMSBOX UND MODULARES WACHSTUMSBOXSYSTEM
CAISSE DE CROISSANCE EMPILABLE ET SYSTÈME DE CAISSES DE CROISSANCE MODULAIRE

(43) Date of publication of application: 11.06.2025
(73) Proprietor: NTFE GmbH, 87675 Rettenbach a. Auerberg (DE)
(72) Inventor: ENGHOF, Jacqueline, 82237 Wörthsee (DE)
(74) Representative: MERH-IP Matias Erny Reichl Hoffmann Patentanwälte PartG mbB
(86) International application number: PCT/EP2022/072055
(87) International publication number: WO 2024/027923

(56) References cited:
- EP-A1- 0 445 320
- EP-A1- 2 567 620
- WO-A1-2015/053542
- CN-A- 110 402 807
- KR-B1- 101 442 457
- KR-Y1- 200 436 615
- US-A1- 2022 061 232

## Description

### Background

The present application relates to a "grow box system", i.e., a system comprising one or more modular, stackable boxes for use in so-called "indoor farming" for plants and proteins (e.g., insects). Indoor farming system is a new way of intensively using space for growing plants or producing proteins.

CN 110 402 807 A discloses a cultivation box comprising a tray; a water tank formed independently of the tray and defining a cavity for containing liquid for plant growth, the water tank being placed on the tray; and a support having a first a first supporting end and a second supporting end opposite to the first supporting end, the first supporting end being inserted into the upper part of the tray, the second supporting end being suitable for being inserted into the lower part of the tray of another cultivation box, and the supporting member helping the water tank positioning on the tray.

### Summary

In a broad aspect, the present disclosure relates to a stackable grow box for an indoor farming system for growing plants or producing proteins. In addition to the advantage of saving space, this new technology enables to farm in whatever climatic conditions in every season in a very sustainable way with the advantage in dramatically reducing the needs of water and other resources.

The invention is set out in the appended claims.

The stackable grow box comprises an upper part, a middle part and a lower part. The lower part comprises a tray to carry a substrate and/or plants or insects (proteins) to be grown in the box. An optional insert plate may be provided in the tray to assist positioning plants (or seedlings) in the tray. The middle part interlinks the upper part and the lower part and defines the distance between the upper and lower parts.

The upper part and the lower part comprise corresponding fluid coupling means configured to provide an automatic fluid coupling when an upper grow box is arranged on top of a lower grow box. The fluid coupling means of the upper and lower parts mate and couple when the upper box is lowered onto the lower box, and provide a tight coupling between the boxes. Thus, boxes can be stacked on top of each other and a fluid coupling is automatically achieved so that a fluid can flow through the stack of boxes, preferably from top to bottom, without requiring any additional manual tubing. The fluid may be water and/or a nutrient solution that supports growth of the plants or insects.

The box further comprises fluid handling means configured to dispense fluid received from the fluid coupling means of the upper part to the plants or insects in the tray, and for collecting excess fluid from the tray and passing it to the fluid coupling means of the lower part. Fluid may be dispensed in the lower part by respective openings of the lower part, or from openings arranged in the upper part in form of fluid drops, streamlet or spray. Thus, a controlled supply and drain of the fluid in the tray is attained that supports growth of the plants or insects. When boxes are stacked, fluid flows, e.g., from top to bottom, through the stacked boxes and supplies the plants and insects in the boxes with water and/or nutrient solution. Further resources for supporting growth of the plants or insects may be supplied as set out below.

In embodiments, the upper part and the lower part may comprise corresponding power coupling means configured to provide an automatic coupling of electrical power when an upper grow box is lowered and arranged on top of a lower grow box. Thus, stacking of boxes is facilitated and electrical power is provided to all stacked boxes without any manual assistance. The electrical power may be used to power additional devices arranged at the boxes that support growth of the plants or insects.

In embodiments, the upper part and the lower part may comprise corresponding positioning means configured to provide an automatic position alignment when an upper grow box is lowered on top of a lower grow box. For example, a conical pin and a corresponding opening may be provided to assist correct alignment of stacked grow boxes. Thus, stacking of boxes is facilitated without manual assistance in aligning the boxes and attaining a straight stack.

The fluid coupling means of the lower part may comprise a valve, e.g., a check valve, that opens the fluid coupling when the box is arranged on top of another lower box. Thus, spillage of fluid is prevented when the box is lifted or not sitting on top of another box, and an automatic fluid coupling is achieved.

The fluid coupling means of the upper part comprises 2 (or more) fluid inlets arranged at opposite positions of the upper part. For example, the fluid inlets may be arranged on the upper surface of the upper part at opposite corners thereof. In addition, a fluid outlet that is coupled with the fluid handling means may be provided at the upper part. The 2 fluid inlets may be interconnected so that fluid entering at any of the 2 inlets exits the upper part at the fluid outlet. Thus, boxes may be arranged in positions rotated by 180° and still a fluid coupling with the lower box(es) is achieved.

The middle part of a box comprises a plurality of columns that links the upper part and the lower part. Preferably, 4 columns are provided at or near the corners of the box to link the upper and lower parts at a defined distance between these. Thus, an empty head space is provided between the upper and the lower part of the box that allows growth of the plants and insects. The head space may be used to supply light, air and/or ventilation. Depending on the application, the space between upper and lower part of the box may also be (partly) closed by adding one or more side walls that may be attached to the columns and/or the upper and lower parts.

The power coupling means may provide for an electric connection between a column of the lower box and a corresponding column of the upper box (or between 2 or more respective columns). The electrical coupling may be by means of a plug and a socket, or by providing direct electrical contact between the columns. Preferably, the columns are made of a conductive material, or comprise electrical wires or conductors. Electric power between the upper and the lower part of a box may be conducted via the at least one column. For example, 2 columns may be respectively applied with a supply voltage and ground, or a positive and a negative supply voltage. Thus, power distribution in a stack of boxes can be achieved without requiring manual wiring of the boxes.

In embodiments, the box may comprise one or more support devices for supporting the growth of the plants or insects in the box. Examples of support devices are a camera, a lighting system, a fluid filling level monitoring system, a growth monitoring system, a ventilation system, and a humidification system. The support device may be mounted on a bar that is affixed to the at least one column. The column(s) may supply electrical energy to the support device. For example, the bar may be in electrical contact with 2 columns that supply power, and comprise wires or conductors to carry current from the columns to the supply device.

The fluid handling means of the box may comprise a fluid inlet that is coupled with the fluid coupling means of the upper part to receive the fluid. The coupling with the fluid coupling means of the upper part may be via a tube or a hose. The fluid handling means may further comprise fluid discharge means arranged in a frame of the lower part for dispensing received fluid to the substrate and/or plants or insects in the tray. For example, the frame of the lower part may comprise one or more openings that are coupled with the fluid inlet, for discharging the fluid into the tray.

The fluid handling means may comprise a number of holes arranged on different levels in the frame of the lower part for receiving excess fluid from the tray and passing it to the fluid coupling means of the lower part. The holes of a same level may be connected via a respective fluid passage provided in the frame to collect the fluid that has achieved (or exceeds) the corresponding level in the tray.

The box may further comprise filling level control means for controlling the fluid filling level of the tray by selectively opening or closing the coupling between a respective fluid passage and the fluid coupling means of the lower part. For example, a plug may close a respective fluid passage, or the linkage of a fluid passage with the fluid coupling means so that fluid that enters the corresponding holes cannot run off. By closing the passages corresponding to holes arranged on lower levels, only fluid exceeding these levels is drained from the tray and the filling level is controlled. Alternatively, level-controlled valves may be provided on the inlet or outlet side of the fluid supply of the tray.

The fluid discharge means of the fluid handling means may be arranged at one side of the frame of the lower part and the holes may be arranged at the opposite side of the frame of the lower part. Thus, the fluid flows in the tray or disperses in the substrate from the one side to the opposite side of the lower side.

The fluid coupling means of the lower part may be arranged at a position that is vertically below the fluid coupling means of the upper part. Thus, when boxes are stacked, the positions of fluid inlet and outlet of the boxes match, and fluid inlet and outlet can mate. When the fluid outlet of the fluid handling means is arranged at one side of the frame and the holes are arranged at the opposite side of the frame, a channel in the frame couples the holes and fluid passages with the fluid coupling means of the lower part, i.e., the fluid is returned to the side of the frame where the fluid coupling means are positioned.

The box comprises engagement openings for engagement with a lifting gear to lift and carry the box. The engagement openings are positioned on the top surface of the upper part of the box so that the lifting gear (e.g., a crane or robot) can engage therewith from above. As mentioned above, positioning means facilitate correct placement and alignment of a box on top of another box.

In embodiments, the box may comprise a lid covering the upper part of the box. Preferably, the lid is arranged within the frame of the upper part and does not vertically extend beyond the upper part so that the top surface of the upper part is not covered by the lid. Hence, fluid and power coupling are not affected by the lid.

One or more optional support devices for supporting the growth of the plants or insects may be mounted to the lid. The support devices may be provided with electrical power, e.g., via wires or conductors attached to the lid.

According to another aspect, a modular plant or insect grow system is disclosed. The system comprises at least one stacked box as disclosed above, and a docking station that serves as a steady basis for the stacked box(es). The docking station is configured to carry a first box of the at least one stacked box. For this, the docking station has a similar form or shape as the box(es) so that the first box fits well on top of the docking station.

The docking station comprises fluid coupling means configured to interact with the fluid coupling means of the lower part of the first box to provide an automatic fluid coupling when the first box is lowered on top of the docking station. The fluid coupling means of the docking station may be similar to the fluid coupling means of the upper part of the box(es) to provide an automatic tight coupling.

In embodiments, the docking station may comprise power coupling means configured to provide an automatic coupling of electrical power with the first box, similar as with the power coupling between boxes.

Furthermore, the docking station may comprise positioning means similar to the boxes, to assist the correct alignment of the boxes on top of the docking station. The docking station may further comprise a fluid sump for collecting the fluid running down from the first box (and from the other boxes via the first box).

The boxes and the docking station may share further features as disclosed in this document to form a modular grow system for plants or insects. The system may comprise additional elements such as a fluid reservoir that may be positioned on the top of the stack of boxes to supply fluid to the topmost box from which it flows down through the other stacked boxes. Other modifications may become apparent to the skilled person from the study of the present disclosure.

### Brief Description of Figures

Example embodiments of the present disclosure will become apparent during the course of the following discussion and by reference to the accompanying drawings. Example embodiments of the disclosure will now be described, by way of example only, with reference to the accompanying drawings in which:
Fig. 1 is a perspective view of an example for a box of the disclosed system.
Fig. 2 is a perspective view of an example upper part of a box.
Fig. 3 is a perspective view of an example lower part of a box.
Fig. 4 shows an example of a box with additional devices.
Fig. 5 is a perspective view of an example lower part having an insert plate for seedlings or plants.
Fig. 6 is a sectional view of an example of a box.
Fig. 7 is a perspective view of an example docking station.
Fig. 8 is a perspective view of an example system comprising a docking station and a box.
Fig. 9 is a sectional view of an example system comprising a docking station and a box.
Fig. 10 shows an example of light strips fitted to the lid of a box.
Fig. 11 is another perspective view of an example box.
Fig. 12 is another perspective view of an example box.
Fig. 13a, b illustrate schematically an example for a fluid coupling between 2 boxes.
Fig. 14 illustrates schematically an example power supply.
Fig. 15 is a perspective view illustrating the stacking of boxes.
Fig. 16 illustrates a cluster of boxes.
Fig. 17 is another perspective view of an example box.

### Detailed Description

The present document discloses a "grow box system", i.e., a system comprising one or more modular, stackable boxes for use in so-called "indoor farming" for plants and proteins (e.g., insects). The system provides the necessary media supplies for the growth of the plants or proteins in a flexible way based on a modular box approach. The term "media" shall mean any resources that are necessary for the growth of the plants or proteins such as water, nutrient solution, electricity, air, etc.

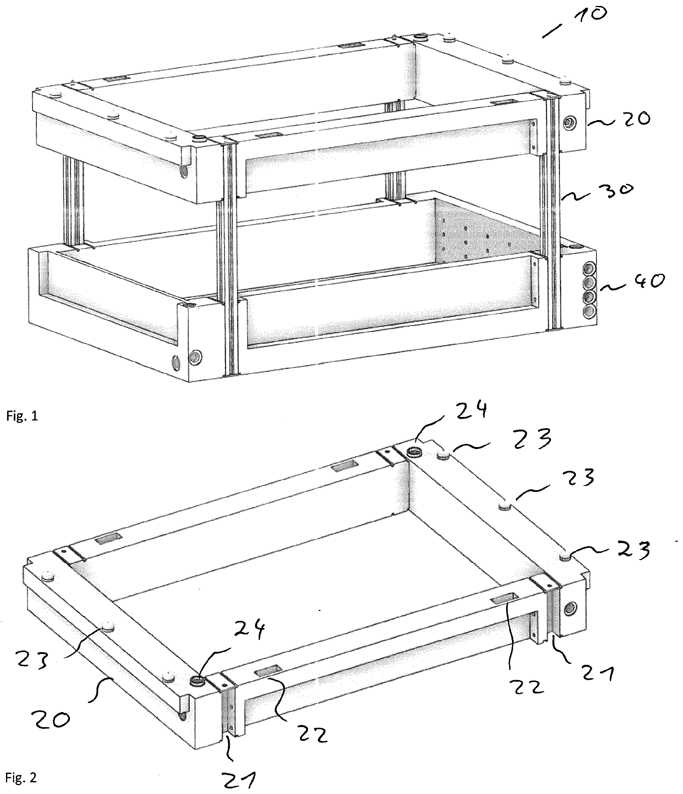

Fig. 1 shows a perspective view of an example embodiment for a box 10 of the disclosed system. The box 10 can be used open as well as closed, individually or in a system comprising a number of boxes and docking station. The boxes 10 of the system 1 are stackable by placing them on top of each other, and may be arranged side-by-side to form large clusters of boxes. A box 10 comprises an upper part 20, middle part 30 and lower part 40. The form and shape of the parts is specifically designed to be stackable, easy to produce and automize the stacking operation.

The upper part 20 may accommodate basic stacking functions via positive (i.e., protruding) positioning means 23 (see Fig. 2) that fit into the lower part 40 of another box, e.g., by extending into a negative positioning means (e.g., an aperture) of the lower part 40.

Furthermore, light and media supply are also installed or can be installed in the upper part 20 (depending on the particular application).

The middle part 30 essentially comprises columns 31, which are used on the one hand for structural stability to connect upper and lower part, and on the other hand for energy supply (described in detail below). The column height/box height can be freely selected via the length of the columns. Furthermore, additional sensors, cameras or lights may be installed to the box 10 via the columns 31, even at a later stage. The columns 31 may further provide for the energy supply of these support devices. In the depicted example, 4 columns 31 are provided, approximately at the 4 corners of the box.

The lower part 40 of the box is used to hold plants, or insects. The lower part 40 may comprise counterparts to the positioning means 23 of the upper part 20 to assist in aligning and stacking of boxes.

The lower part is designed to align with the upper part of the columns of the lower box. Thus, the lower part 40 may be designed such that the columns of an upper box are arranged on top of and have contact with the columns of a respective lower box. Thus, electrical contact between respectively aligned columns of these boxes may be provided. For this, the columns 31 may vertically extend through the lower part 40 up to its lower surface, and may vertically extend through the upper part 20 up to its upper surface. Optionally, plugs and sockets may be provided at the columns to improve electrical contact. For example, a protrusion at a longitudinal upper end of a column and a corresponding receptable at the longitudinal lower end of a corresponding column of an upper box may be provided (or vice versa). In variations, the columns do not completely extend vertically through the lower and/or upper parts so that the columns are not accessible on the upper side of the upper part 20 and/or the lower side of the lower part 40. For example, the top of a column may be covered by a portion of the upper part and/or the bottom of a column may be covered by a portion of the lower part. In this case, the electrical contact between columns that are arranged on top of each other is provided by other means. For example, a protrusion or extension pin of a column may extend through the covering portions and make contact to the upper (or lower) column. As mentioned, power supply to boxes may be provided by the columns carrying electrical current. For example, a first column may be assigned to ground and the other column on the same long side of a box may be assigned to a supply voltage (AC or DC). Alternatively, the first column may carry a negative DC voltage and the second column a positive DC voltage (or vice versa). If the voltage assignment on the other long side of the box is reversed (i.e., diagonal columns have the same voltage), boxes may be stacked in both orientations (i.e., flipped by 180°). Alternatively, different supply voltages may be applied to the columns, to power different kinds of support devices.

In addition, there is a fluid connection between the upper part 20 of a lower box and the lower part 40 of an upper box to provide a fluid communication between the boxes so that a nutrient solution and/or water can flow through the interconnected boxes from top to bottom.

The design of the boxes and in particular their interlocking mechanisms provides for a top-down or bottom-up system for the various necessary media supplies (water, electricity, air, ... etc.).

The box 10 may also be closed by inserting so-called side walls if required (see below). In mass production, boxes may be integrally made out of one piece, e.g., by plastic injection molding or other techniques. Alternatively, the frame structures of the box may be formed by aluminum casting.

Fig. 2 shows a perspective view of an example embodiment of an upper part 20 of a box 10. The upper part 20 is a frame construction that can be closed with an optional lid (not shown). In the frame, there are lateral recesses 21 for the four columns 31 as connection to the lower part 30. The recesses 21 may vertically extend completely through the frame of the upper part so that mounted columns are accessible from top.

Alternatively, the recesses 21 are closed on the top surface of the frame and mounted columns do not completely extend vertically through the frame.

In the depicted example, the upper part frame is designed to save material in the side structure by reducing volume, e.g., of the longitudinal supports that is not necessary for structural stability. The recesses formed by the reduced dimension in the lateral dimension of the supports further facilitates frame production, e.g., by molding.

Further, there are engagement openings 22 in the upper part 20 for engagement with a lifting gear, which can be used for lifting and setting down a respective box (e.g., for automatic lifting in the case of an automated system). The lifting gear may have corresponding hooks that engage with the engagement openings 22 (e.g., by spreading after being inserted) to provide a secure locking with the box for lifting and moving the box.

On the top surface of the upper part 20 may be positioning means 23 (e.g., conical pins) for engagement with corresponding counterparts (e.g., recesses) in a lower surface of the lower part 40 of an upper box, for aligning and fixing the boxes. The arrangement and the number of positioning means 23 may vary depending on the intended application.

The supply or inflow of nutrient solution/water is provided through one or more fluid inlets 24 arranged on the top surface of the upper part 20, e.g., in diagonal corners of the frame. Through a channel in the upper part 20 (not shown), the inlets 24 are connected and the box can also be placed rotated by 180°. This does not affect either the current or the nutrient solution/water supply.

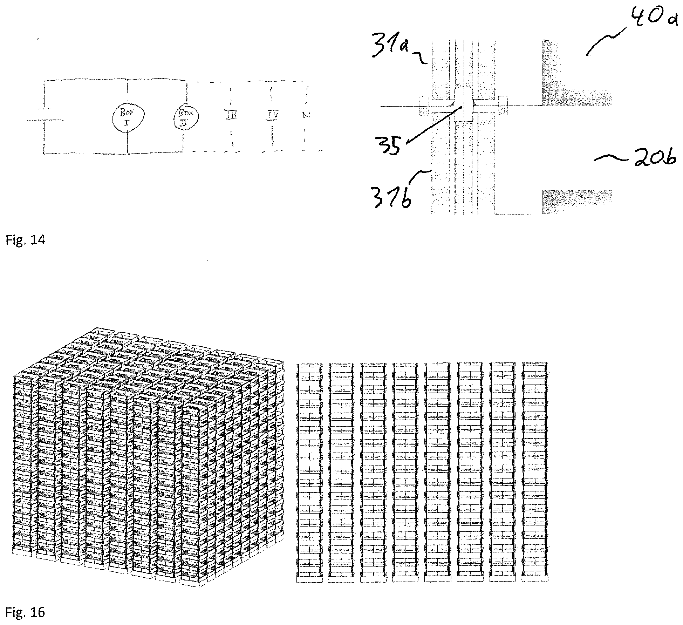

The optional lid of the upper part 20 can have several functions. In general, it may serve as an attachment point for light sources and, if necessary, additional water tanks and/or spraying systems for humidification (see below). Preferably, the lid is arranged inside of the frame structure and does not extend vertically beyond the frame structure so that it does not interfere with the mechanisms arranged on the top surface of the upper part 20. The middle part 30 comprises 4 columns 31 connecting the upper and lower parts. The length of the columns 31 is freely selectable (e.g., by cutting the columns respectively). One or more of the columns 31 may serve as conductors for power supply and forwarding current between the boxes. For this purpose, columns 31 may be made of conductive material. Alternatively, cables or conductors may be arranged within the columns 31. In order to ensure current connection to the respective upper/lower box, the columns 31 may be equipped with optional plugs which can be inserted into the lower/upper side of the respective upper/lower box or its column(s) so that an electrical contact is made. Thus, a direct electric contact between columns of an upper and a lower box (with or without plugs) may provide for the power supply of the boxes. For example, a plug may be positioned at the upper end of a column 31 that may interact with a respective socket on the lower end of a column of a box sitting on top (see Fig. 14).

The accuracy or positioning of the respective boxes is ensured via corresponding positive positioning units in the upper part and lower part of the box.

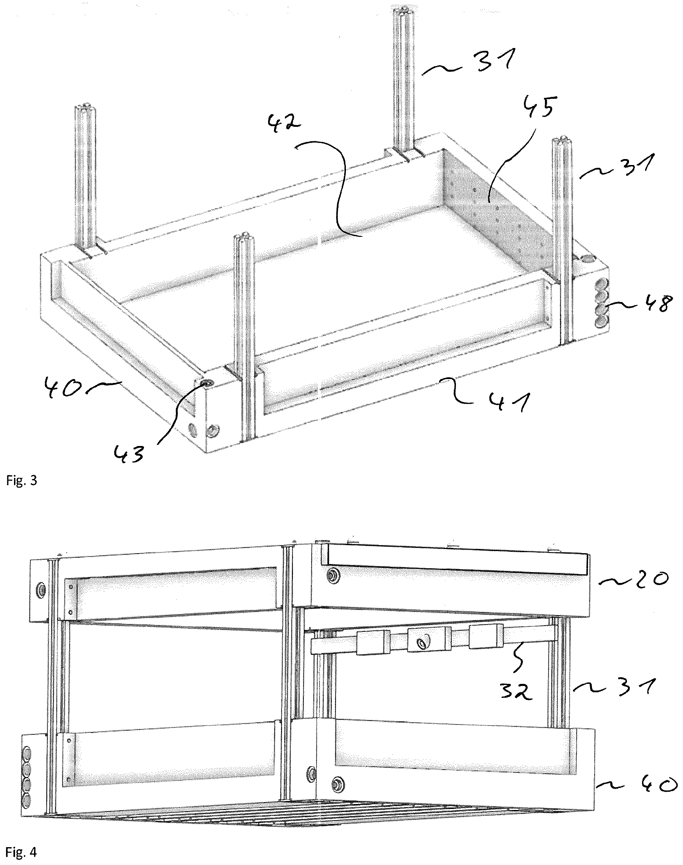

The columns 31 may be further employed for fixation of optional side walls and/or additional support devices, e.g., for ventilation, sensors, etc. Fig. 4 shows an example of a box 10 where additional devices are arranged on a bar 32 that is mounted to 2 columns 31. Power supply for the additional devices may be provided via the bar 32 (e.g., by wires) having electrical contact with the current-carrying columns 31. The columns may have nuts on their sides to support mounting of the bar 32,

Fig. 3 shows a perspective view of an example embodiment of a lower part 40 having attached columns 31 of the middle part 30. The lower part 40 is primarily used to hold plants or proteins (e.g., insects). Frame 41 and floor plate 42 form a watertight tray to carry the plants or proteins. In embodiments, a substrate is accumulated in the tray and plants or insects are arranged in or on the substrate.

Frame 41 has recesses on the sides, analogous to the upper part, for fastening the columns 31. Again, the recesses may vertically extend completely through the frame of the lower part so that mounted columns are accessible from below. Alternatively, the recesses are closed on the bottom surface of the frame and mounted columns do not completely extend vertically through the frame.

Similar as for the upper part, the frame may be designed to save material by reducing volume of the supports that is not necessary for structural stability. The recesses formed by the reduced dimension in the lateral dimension of the supports further facilitates frame production, e.g., by molding.

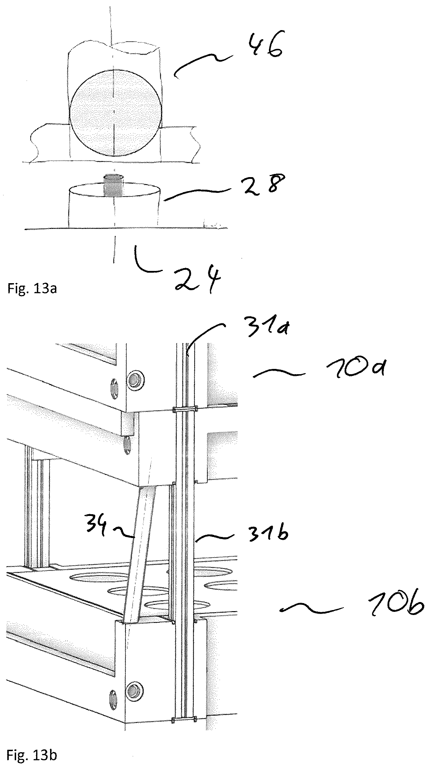

Furthermore, the lower part 40 has a fluid connection with the upper part 20 for the supply of fluids (nutrient solution/water). For example, a connection tube or hose (see Fig. 13b) may connect a fluid outlet 25 of the upper part 20 with an inlet 43 of the lower part 40 to allow a fluid flowing from the upper part to the lower part. The inlet 43 is connected with a supply opening of the frame 41 (not shown) that discharges the fluid into the tray.

On the opposite side of the frame 41, there may be a number of holes 45 with which the filling level of the lower part can be preset. Alternatively, the filling level can also be set with a control valve controlling the fluid supply. In this case, this must be programmed in advance to the respective fill level. The nutrient solution/water runs into the box and is drained off on the opposite side. This ensures a constant flow in using gravity. The nutrient solution/water flows via a check valve-controlled fluid outlet 46 to the next (lower) box or into a drain. When a box is lifted, the check valve in the lower part 40 closes and ensures that no further nutrient solution escapes.

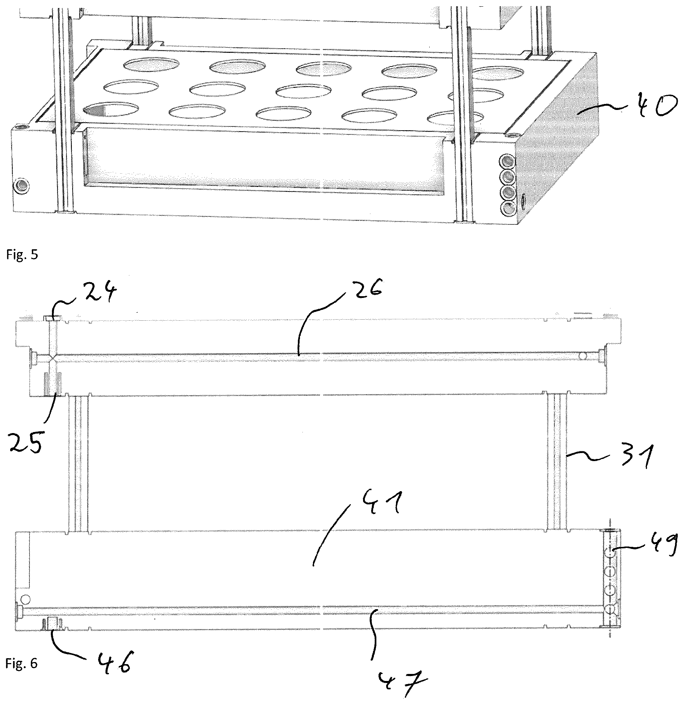

Fig. 5 shows a perspective view of an example embodiment of a lower part 40 having an insert plate for seedlings or plants, the insert plate having a grid of openings. The seedlings or plants can be arranged at the fixed positions of the grid so that they do not interfere with each other.

Fig. 6 shows a sectional view of an example embodiment of a box 10. One can see a fluid inlet 24 and a fluid outlet 25 in the upper part 20. A connection tube or hose may be attached to the fluid outlet 25 in order to supply fluid to the fluid inlet 43 of the lower part 40. An optional fluid channel 26 may be provided in the upper part 20 to connect the fluid outlet 25 with another (optional) fluid inlet 24 arranged in a diagonal corner of the upper part 20. This allows for a 180-degree rotated position of an upper box on a lower box while maintaining the fluid communication between these boxes.

A fluid channel 47 in the lower part 40 returns the fluid from the side of the frame 41 where the holes 45 are arranged for collecting the fluid from the tray, to the side of the frame 41 where the outlet 46 is provided. In the depicted example, the outlet 46 of the lower part 40 is positioned on the same side and corner of the box where the inlet 24 of the upper part 20 (or one of the inlets 24) is provided. This allows for a fluid coupling between boxes when the boxes are stacked. If 2 diagonally arranged fluid inlets 24 are provided in the upper part, boxes may be stacked in any orientation, i.e., rotated by 180 degrees. Alternatively, or in addition, 2 fluid outlets arranged in diagonal corners of the box may be arranged for to provide flexibility in stacking of boxes. Mating couplers are provided at the inlet 24 and outlet 46 to improve locking and tightness of the fluid coupling. In addition, a check valve opening when the box is positioned on a lower box may be provided at the outlet 46.

As already mentioned, the fluid is drained from the tray via holes 45. For conducting and regulation of the level of nutrition/ water in the tray, the holes 45 are arranged on different levels, and holes of one level are interconnected via a respective horizonal passage 48. A vertical passage 49 connects the horizontal passages 48 with the channel 47. Openings in the frame 41 are provided where horizontal passages 48 meet the vertical passage 49. In order to preset the filling level of the tray, the junction between individual horizontal passages 48 and the vertical passage may be closed via plugs inserted into selected openings. The dimension of the plugs is such that the plugs fill out the intersection between horizontal and vertical passages, thereby closing the fluid passage. For example, by inserting plugs in all but one openings of the horizontal passages 48, a horizontal passage and its corresponding holes at a designated filling level are selected, while fluid drainage through non-selected holes is prevented. Of course, it is also possible to only close horizontal passages 48 that are below the desired filing level. In order to avoid fluid spillage, openings may be covered by caps.

In order to facilitate production of the upper and lower frames, channels 26 and passages 48, 49 may be formed as through holes and their ends closed with caps.

The system may further comprise a docking station 50. The docking station can be used for the basic supply of each stack with power and nutrient solution/water. If necessary, additional sensors and also other applications required for the application can then be subsequently installed.

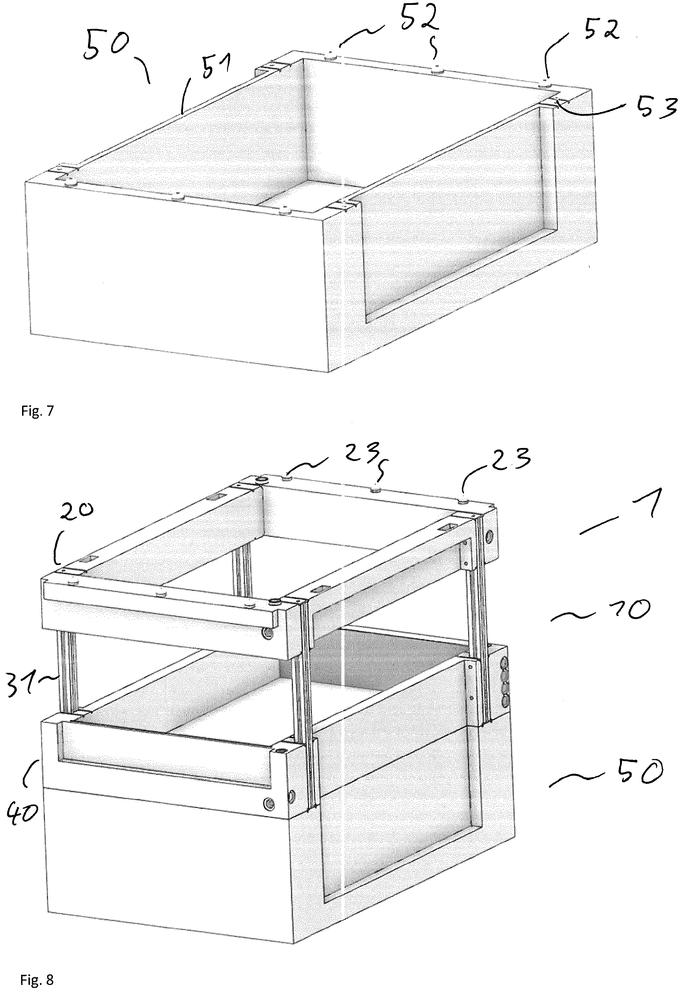

Fig. 7 shows a perspective view of an example embodiment of a docking station 50. The docking station has a frame 51 and a bottom plate to form a tank for the fluid. Contour, shape and dimensions of the docking station frame 51 may correspond to the frame 41 of a box. In particular, the upper surface of the docking station 50 may match the contour, shape and dimensions of the lower surface of the box so that the box fits on top of the docking station and can rest there safely.

The docking station is a frame structure that serves as a base for stacking multiple boxes. Several docking stations can be connected to each other to collect and/or drain run-off nutrient solution/water.

As with a box 10, the docking station 50 has positive positioning means 52 on its upper surface that mate with the negative positioning means (e.g., recesses) of the lower part 40 of a fitting box to assist alignment of the box 10 on the docking station 50.

Electrical connectors 53 are arranged on the upper surface of the docking station 50 to provide power to the box 10. For example, the connectors 53 may contact one or more of the columns 31 to provide power to the box 10. For this, the connectors 53 are positioned at locations on the upper surface that correspond to the columns 31 and allow electrical contact therewith. As with boxes, the connectors 53 may comprise plugs that interact with respective sockets on the lower end of the columns 31.

As mentioned above, the columns 31 may vertically extend through the frame 41 of the lower part 40 so that they are accessible from below by the connectors 53. Alternatively, the connectors 53 do not directly contact the columns 31 but provide connection with electrical contacts of the lower part 40. Electric currency is then forwarded to the columns 31, or to cables or conductors within the columns.

Further, the docking station may be equipped with a drain or sump to collect run-off nutrient solution/water and convey it to a treatment station if needed. It is also possible that the nutrient solution / water is pumped back to the highest box in the stack via a pump and filter system, thus creating a circulation system in using gravity.

The docking station may comprise draining means similar to the fluid inlet 24 of the upper part 20 in order to interact with the fluid outlet 46 of the lower part 40 of a fitted box 10 and to collect the fluid drained from a box (not shown in Fig. 7). In case of a check valve arranged in the fluid outlet 46, the draining means activates/opens the check valve when the box is lowered and fitted on top of the docking station 50 so that the fluid can leave the box and flow into the docking station. 2 draining means arranged at diagonal corners of the upper level of the docking station 50 may be provided so that a box can be fitted on top irrespective of its orientation (i.e., rotated by 180 degrees) and one of the draining means can interact with the fluid outlet of the box.

Furthermore, the docking station may contain a control system for the regulation of attached sensors or a data logger for data recording. This control system can be as well extern to the docking station. The docking station may also have another sensor for monitoring any malfunctions in the irrigation or nutrient solution circuit. This senor strikes when nutrient solution overflows from a stack and does not flow through the designated piping system.

Fig. 8 shows a perspective view of an example embodiment of a system 1 comprising a docking station 50 and a fitted first box 10. Further boxes may be stacked on top. The alignment of these further boxes may be facilitated by the positioning means 23 so that a straight stack of boxes is achieved. Power supply for the boxes can be accomplished via the columns 31.

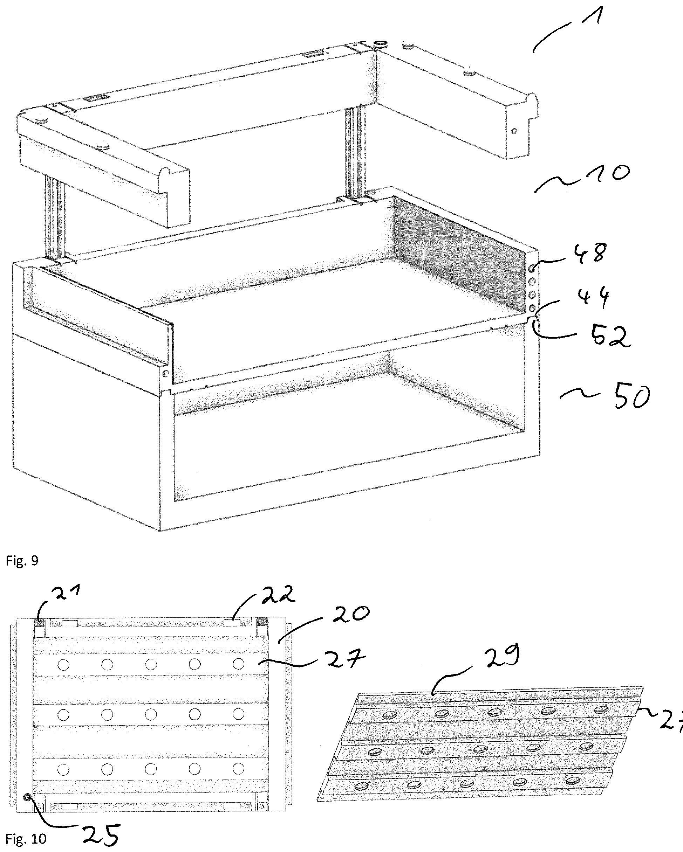

Fig. 9 shows a sectional view of an example embodiment of a system 1 comprising a docking station 50 and a fitted first box 10. Positioning means 52 of the docking station 50 engage with corresponding negative positioning means 44 (e.g., recesses) in the lower part 40 of the box. The figure further shows the horizontal passages 48 that collect fluid from the corresponding holes.

Each individual box can be equipped with appropriate lighting (e.g., UV-LED) to ensure optimal growth of the plants or animals. If necessary, the lighting can be centrally or individually controlled and adjusted in intensity and/or color spectrum to influence taste, growth or other criteria.

The lighting can be installed as an individual light or, for example, as a light strip in the lid of the box. Fig. 10 shows an example of light strips 27 as an example for a support device fitted to the lid 29 of an upper box part 20. The left figure shows the upper part 20 closed by the lid 29 from below and the right figure shows a perspective view of the lid 29. In the left figure, one can see the frame of the upper part 20 from below, including the recesses 21 for holding the four columns 31, the engagement openings 22 for engagement with a lifting gear, and the fluid outlet 25.

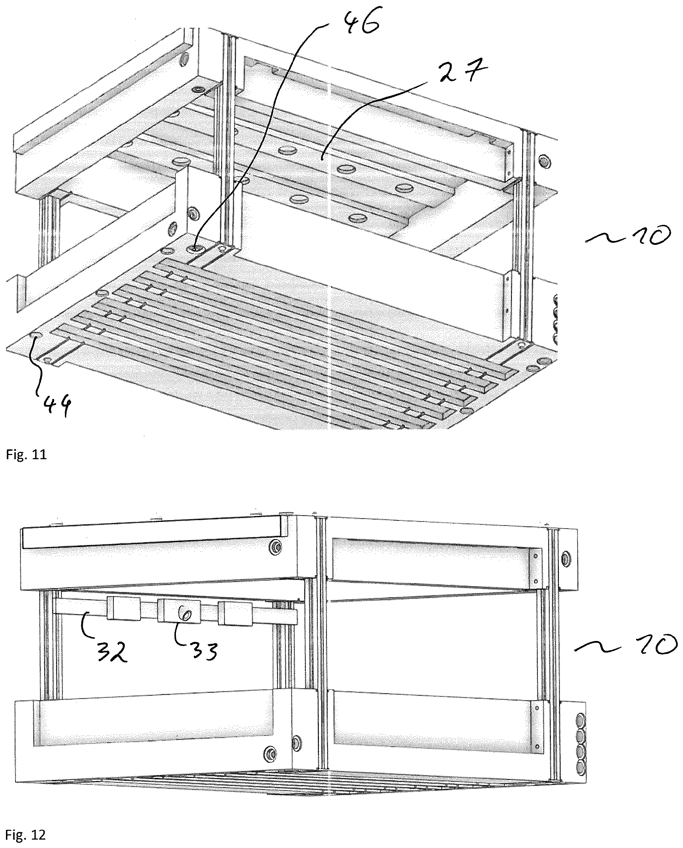

Fig. 11 shows another perspective view of an example embodiment of a box 10 having a lid 29. A lighting system 27 is provided in the lid 29. In this view from below, one can further see the recesses 44 and the fluid outlet 46 in the lower part 40 of the box.

A box may be equipped with a fan or other ventilation system if required. Like other support devices, fans can also be retrofitted to the box. Either these are permanently on until a box is taken down from the system, or can be controlled individually via remote control using wireless communication such as Bluetooth or WiFi.

Fig. 12 shows another perspective view of an example embodiment of a box 10. A bar 32 or similar fixation system can be fixed on the box to mount a ventilation system, a camera, light or sensors. The bar 32 may be attached to columns 31 which can provide power supply via the bar to the support devices. For example, a camera system 33 can be installed in the lid 29 and/or via the bar 32 on the columns 31, if required. Depending on the application and settings, the camera can either send images at given time intervals to a memory location or be actively switched on as required.

Further, a box may be equipped with an optional humidification system. The humidification system may be an electric pump with an integrated tank, which may be controlled via remote control (e.g., Bluetooth or WiFi) and distributes moisture from the top of the box either at predefined intervals or on demand.

Irrigation can be done in different ways depending on the application. Basically, a system comprising several boxes may operate by feeding water/nutrient solution from the top box and uses gravity to make the water/nutrient solution flow from the top box to the bottom box and finally to the docking station. As described before, a filter and pump system can also be connected to the docking station, which then treats unused water/nutrient solution accordingly and pumps it to the top box via a pipe system.

Fig. 13a illustrates schematically an example for a fluid coupling between 2 boxes. When lifting a box 10, a check valve in the bottom outlet 46 of the box prevents water/nutrient solution from escaping. A protrusion 28 of the inlet 24 on top of the box's upper part 20 opens the valve when the upper box is lowered and fitted.

Fig. 13b illustrates schematically the coupling of 2 boxes: an upper box 10a that sits on a lower box 10b, the columns 31a, 31b aligned to make electrical contact between the 2 boxes. A tube 34 connects the fluid outlet 25 of the upper part 20 with the inlet 43 of the lower part 40 to allow the fluid flowing from the upper part to the lower part.

The power supply for the boxes is designed in such a way that each time a next box is placed on top of the previous one, it is supplied with power in parallel. There are 2 possibilities: 1^{st} alternative would be a fixed wiring via the electrical connections provided by the columns 31 (see Fig. 13b). 2nd alternative would be a battery module, which can be placed on the top box, for example, or is present in each box and then takes over the box power supply for support devices arranged in the box. Of course, both options can be combined.

Fig. 14 illustrates schematically an example power supply that provides a parallel connection of boxes during stacking and enables "energizing" of each box after stacking. The left figure illustrates schematically the electrical arrangement in parallel of the boxes, while the right arrangement shows an example for an electrical connection between an upper box 10a and a lower box 10b via their respective columns 31a and 31b. As already mentioned, column 31a is received by the lower part 40a of the upper box 10a, and column 31b is received by the upper part 20b of the lower box 10b. A pin 35 protruding either from the column 31a of the upper box 10a or column 31b of the lower box 10b electrically connects the columns 31a, 31b. In order to avoid damage to the pin 35, it may be movable in its longitudinal direction, e.g., popped out by a spring. In the shown example, electricity is conducted by a conductor (e.g., a wire) within the columns 31a, 31b. Alternatively, the columns itself may be conductive. Of course, electrical power may be provided by more than 1 column and e.g., 2, 3, or even all 4 columns may contribute to power supply.

Box stacking may begin with the installation of a docking station 50, which is the base for media supply and disposal. Several boxes 10 can be stacked on a docking station 50 and supplied with media. The different parts of the box (described in the respective points above) are used here for structural stability and for interconnecting several boxes in a stack. Boxes can be stacked both manually and automatically (via a robot).

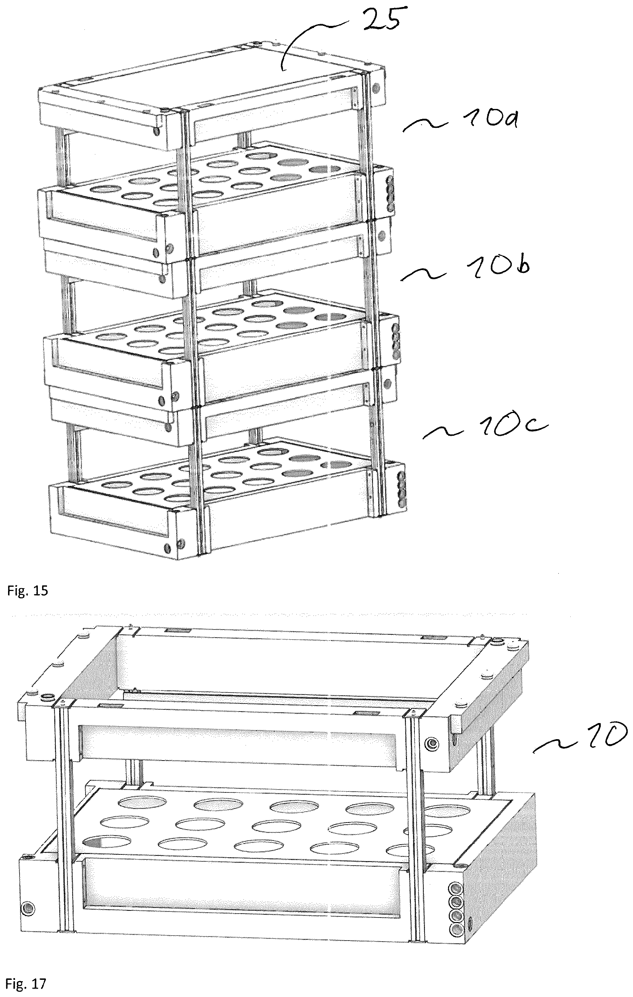

Fig. 15 is a perspective view illustrating the stacking of boxes 10. The boxes 10a, 10b, 10c are aligned via their positive positioning means 23 and their corresponding negative positioning means 44. The possible height of a stack depends, among other things, on the condition of the floor (evenness, load-bearing capacity, slope). In the example stack of Fig. 15, a lid 29 is provided for the top box, and the boxes 10 are equipped with insert plates for seedings or plants.

Several boxes can be stacked on top of each other and also next to each other. Fig. 16 illustrates a cluster of boxes that are stacked vertically and stacks of boxes are arranged next to each other in a rectangular grid. The left side of the figure is a perspective view showing a cube of boxes and the right side is a side view of the box cube.

A box can be loaded both by removing its lid (if provided) or by so-called slide-in compartments/bottoms (see Fig. 17).

The insert plates / compartments offer the possibility of optimal loading of each box with appropriate spacing of each plant, so that in turn an optimal growth can be ensured. Loading with seedlings may be done manually or also automated with a robotic solution / automation.

## Claims

1. Stackable grow box (10) comprising an upper part (20), a middle part (30) and a lower part (40), wherein the lower part (40) comprises a tray to carry plants or insects to be grown in the box, the middle part (30) comprising a plurality of columns (31) for interlinking the upper part (20) and the lower part (40),
wherein the upper part (20) is a frame having lateral recesses (21) to receive the columns, and having engagement openings (22) on the top surface of the upper part (20) for engagement with a lifting gear to lift the grow box (10), wherein the upper part (20) and the lower part (40) comprise corresponding fluid coupling means configured to provide an automatic fluid coupling with a lower grow box (10) when the grow box (10) is arranged on top of the lower grow box (10), the fluid coupling means of the upper part (20) having two fluid inlets (24) arranged on opposite positions of the top surface of the frame of the upper part (20), wherein the fluid coupling means of the upper part (20) and the lower part (40) are configured such that the fluid coupling means of the lower part (40) of the grow box (10) and the fluid coupling means of the upper part (20) of the lower grow box (10) mate and couple when the grow box (10) is lowered onto the lower grow box (10), and
the box comprises fluid handling means configured to dispense fluid received from the fluid coupling means of the upper part (20) to the plants or insects in the tray and for collecting excess fluid from the tray and passing it to the fluid coupling means of the lower part (40).

2. The box (10) of claim 1, wherein the upper part (20) and the lower part (40) comprise corresponding power coupling means configured to provide an automatic coupling of electrical power when an upper grow box (10) is arranged on top of a lower grow box (10).

3. The box (10) of claim 1 or 2, wherein the upper part (20) and the lower part (40) comprise corresponding positioning means configured to provide an automatic position alignment when an upper grow box (10) is lowered on top of a lower grow box (10).

4. The box (10) of any of the previous claims, wherein the fluid coupling means of the lower part (40) comprises a valve that opens the fluid coupling when the box is arranged on top of another lower box (10).

5. The box (10) of any of the previous claims, wherein the fluid coupling means of the upper part (20) comprises a fluid outlet (25) that is coupled with the fluid handling means.
the two fluid inlets interconnected so that fluid entering at any of the 2 inlets exits the upper part (20) at the fluid outlet (25).

6. The box (10) of any of the claims 2 to 5, wherein the power coupling means provide for an electric connection between a column (31) of the lower box (10) and a corresponding column (31) of the upper box (10).

7. The box (10) of claim 6, wherein electric power is distributed between the upper and the lower part via at least one column (31).

8. The box (10) of claim 6 or 7, wherein a support device (33) for supporting the growth of the plants or insects in the box is mounted on a bar (32) that is affixed to at least one column (31) suppling electrical energy to the support device (33).

9. The box (10) of any of the previous claims, wherein the fluid handling means comprise a fluid inlet (43) that is coupled with the fluid coupling means of the upper part (20), and fluid discharge means arranged in a frame (41) of the lower part (40) for dispensing received fluid to the plants or insects in the tray.

10. The box (10) of any of the previous claims, wherein the fluid handling means comprise a number of holes (45) arranged on different levels in the frame of the lower part (40) for receiving excess fluid from the tray and passing it to the fluid coupling means of the lower part (40), the holes of a same level connected via a respective passage (48), the box (10) further comprising filling level control means for controlling the fluid filling level of the tray by selectively opening or closing the coupling between the respective passage (48) and the fluid coupling means of the lower part (40).

11. The box (10) of claim 10, wherein the fluid discharge means are arranged at one side of the frame (41) of the lower part (40) and the holes (45) are arranged at the opposite side of the frame (41) of the lower part (40), the fluid coupling means of the lower part (40) arranged at a position that is below the fluid coupling means of the upper part (40), and the passages (48) coupled via a channel (47) with the fluid coupling means of the lower part (40).

12. The box (10) of any of the previous claims, further comprising a lid (29) covering the upper part (20), wherein an optional support device (27) for supporting the growth of the plants or insects is mounted to the lid (29) and provided with electrical power.

13. A modular plant or insect grow system (1) comprising at least one stacked box (10) according to any of the previous claims, and a docking station (50) that is configured to carry a first box of the at least one stacked box (10), the docking station (50) comprising fluid coupling means configured to interact with the fluid coupling means of the lower part (40) of the first box (10) to provide an automatic fluid coupling when the first box (10) is lowered on top of the docking station (50).

14. The grow system (1) of claim 13, wherein the docking station comprises power coupling means configured to provide an automatic coupling of electrical power with the first box (10).

## Patentansprüche

1. Stapelbare Wachstumsbox (10) mit einen oberen Teil (20), einen mittleren Teil (30) und einen unteren Teil (40), wobei der untere Teil (40) eine Schale aufweist, um Pflanzen oder Insekten zu tragen, die in der Box angebaut bzw. gezüchtet werden sollen, wobei der mittlere Teil (30) eine Vielzahl von Säulen (31) zum Verbinden des oberen Teils (20) und des unteren Teils (40) miteinander aufweist, wobei der obere Teil (20) ein Rahmen ist, der seitliche Aussparungen (21) aufweist, um die Säulen aufzunehmen, und Eingriffsöffnungen (22) an der oberen Oberfläche des oberen Teils (20) zum Eingriff mit einer Hebeausrüstung aufweist, um die Wachstumsbox (10) anzuheben, wobei der obere Teil (20) und der untere Teil (40) entsprechende Fluidkopplungsmittel aufweisen, die dazu konfiguriert sind, eine automatische Fluidkopplung mit einer unteren Wachstumsbox (10) bereitzustellen, wenn die Wachstumsbox (10) oben auf der unteren Wachstumsbox (10) angeordnet ist, wobei die Fluidkopplungsmittel des oberen Teils (20) zwei Fluideinlässe (24) aufweisen, die an gegenüberliegenden Positionen der oberen Oberfläche des Rahmens des oberen Teils (20) angeordnet sind,
wobei die Fluidkopplungsmittel des oberen Teils (20) und des unteren Teils (40) derart konfiguriert sind, dass die Fluidkopplungsmittel des unteren Teils (40) der Wachstumsbox (10) und die Fluidkopplungsmittel des oberen Teils (20) der unteren Wachstumsbox (10) zusammenpassen und koppeln, wenn die Wachstumsbox (10) auf die untere Wachstumsbox (10) abgesenkt wird, und die Box Fluidhandhabungsmittel aufweist, die dazu konfiguriert sind, Fluid, das von den Fluidkopplungsmitteln des oberen Teils (20) empfangen wird, an die Pflanzen oder die Insekten in der Schale abzugeben und überschüssiges Fluid von der Schale zu sammeln und es an die Fluidkopplungsmittel des unteren Teils (40) zu leiten.

2. Box (10) nach Anspruch 1, wobei der obere Teil (20) und der untere Teil (40) entsprechende Leistungskopplungsmittel aufweisen, die dazu konfiguriert sind, eine automatische Kopplung von elektrischer Leistung bereitzustellen, wenn eine obere Wachstumsbox (10) oben auf einer unteren Wachstumsbox (10) angeordnet ist.

3. Box (10) nach Anspruch 1 oder 2, wobei der obere Teil (20) und der untere Teil (40) entsprechende Positionierungsmittel aufweisen, die dazu konfiguriert sind, eine automatische Positionsausrichtung bereitzustellen, wenn eine obere Wachstumsbox (10) oben auf einer unteren Wachstumsbox (10) abgesenkt wird.

4. Box (10) nach einem der vorhergehenden Ansprüche, wobei die Fluidkopplungsmittel des unteren Teils (40) ein Ventil aufweisen, das die Fluidkopplung öffnet, wenn die Box oben auf einer anderen unteren Box (10) angeordnet ist.

5. Box (10) nach einem der vorhergehenden Ansprüche, wobei die Fluidkopplungsmittel des oberen Teils (20) einen Fluidauslass (25) aufweisen, der mit den Fluidhandhabungsmitteln gekoppelt ist,
wobei die zwei Fluideinlässe so miteinander verbunden sind, dass Fluid, das an einem der 2 Einlässe eintritt, den oberen Teil (20) an dem Fluidauslass (25) verlässt.

6. Box (10) nach einem der Ansprüche 2 bis 5, wobei die Leistungskopplungsmittel eine elektrische Verbindung zwischen einer Säule (31) der unteren Box (10) und einer entsprechenden Säule (31) der oberen Box (10) bereitstellen.

7. Box (10) nach Anspruch 6, wobei elektrische Leistung zwischen dem oberen und dem unteren Teil über mindestens eine Säule (31) verteilt wird.

8. Box (10) nach Anspruch 6 oder 7, wobei eine Unterstützvorrichtung (33) zum Unterstützen des Wachstums der Pflanzen oder der Insekten in der Box auf einer Stange (32) montiert ist, die an mindestens einer Säule (31) befestigt ist, die elektrische Energie an die Unterstützvorrichtung (33) liefert.

9. Box (10) nach einem der vorhergehenden Ansprüche, wobei die Fluidhandhabungsmittel einen Fluideinlass (43), der mit den Fluidkopplungsmitteln des oberen Teils (20) gekoppelt ist, und Fluidabgabemittel, die in einem Rahmen (41) des unteren Teils (40) angeordnet sind, zum Abgeben von empfangenem Fluid an die Pflanzen oder die Insekten in der Schale aufweisen.

10. Box (10) nach einem der vorhergehenden Ansprüche, wobei die Fluidhandhabungsmittel eine Anzahl von Löchern (45), die auf verschiedenen Ebenen in dem Rahmen des unteren Teils (40) angeordnet sind, zum Empfangen von überschüssigem Fluid von der Schale und zum Leiten dessen an die Fluidkopplungsmittel des unteren Teils (40) aufweisen, wobei die Löcher einer gleichen Ebene über einen jeweiligen Durchgang (48) verbunden sind, wobei die Box (10) ferner Füllniveausteuermittel zum Steuern des Fluidfüllniveaus der Schale durch selektives Öffnen oder Schließen der Kopplung zwischen dem jeweiligen Durchgang (48) und den Fluidkopplungsmitteln des unteren Teils (40) aufweist.

11. Box (10) nach Anspruch 10, wobei die Fluidabgabemittel auf einer Seite des Rahmens (41) des unteren Teils (40) angeordnet sind und die Löcher (45) auf der gegenüberliegenden Seite des Rahmens (41) des unteren Teils (40) angeordnet sind, wobei die Fluidkopplungsmittel des unteren Teils (40) an einer Position angeordnet sind, die sich unter den Fluidkopplungsmitteln des oberen Teils (40) befindet, und die Durchgänge (48) über einen Kanal (47) mit den Fluidkopplungsmitteln des unteren Teils (40) gekoppelt sind.

12. Box (10) nach einem der vorhergehenden Ansprüche, ferner mit einem Deckel (29), der den oberen Teil (20) abdeckt, wobei eine optionale Unterstützvorrichtung (27) zum Unterstützen des Wachstums der Pflanzen oder der Insekten an dem Deckel (29) montiert ist und mit elektrischer Leistung versorgt wird.

13. Modulares Pflanzenanbau- oder Insektenzucht-System (1) mit mindestens einer gestapelten Box (10) nach einem der vorhergehenden Ansprüche und einer Andockstation (50), die dazu konfiguriert ist, eine erste Box der mindestens einen gestapelten Box (10) zu tragen, wobei die Andockstation (50) Fluidkopplungsmittel aufweist, die dazu konfiguriert sind, mit den Fluidkopplungsmitteln des unteren Teils (40) der ersten Box (10) zusammenzuwirken, um eine automatische Fluidkopplung bereitzustellen, wenn die erste Box (10) oben auf der Andockstation (50) abgesenkt wird.

14. Anbau- oder Zucht-System (1) nach Anspruch 13, wobei die Andockstation Leistungskopplungsmittel aufweist, die dazu konfiguriert sind, eine automatische Kopplung von elektrischer Leistung mit der ersten Box (10) bereitzustellen.

## Revendications

1. Boîte de croissance empilable (10) comprenant une partie supérieure (20), une partie médiane (30) et une partie inférieure (40), dans laquelle la partie inférieure (40) comprend un plateau pour porter des plantes à cultiver ou des insectes à élever dans la boîte, la partie médiane (30) comprenant une pluralité de colonnes (31) pour relier la partie supérieure (20) et la partie inférieure (40),
dans laquelle la partie supérieure (20) est un cadre ayant des évidements latéraux (21) pour recevoir les colonnes, et ayant des ouvertures de mise en prise (22) sur la surface supérieure de la partie supérieure (20) pour la mise en prise avec un équipement de levage pour lever la boîte de croissance (10), dans laquelle la partie supérieure (20) et la partie inférieure (40) comprennent des moyens de couplage de fluide correspondants configurés pour fournir un couplage de fluide automatique avec une boîte de croissance inférieure (10) lorsque la boîte de croissance (10) est agencée sur le dessus de la boîte de croissance inférieure (10), les moyens de couplage de fluide de la partie supérieure (20) ayant deux entrées de fluide (24) agencées sur des positions opposées de la surface supérieure du cadre de la partie supérieure (20),
dans laquelle les moyens de couplage de fluide de la partie supérieure (20) et de la partie inférieure (40) sont configurés de sorte que les moyens de couplage de fluide de la partie inférieure (40) de la boîte de croissance (10) et les moyens de couplage de fluide de la partie supérieure (20) de la boîte de croissance inférieure (10) s'accouplent et se couplent lorsque la boîte de croissance (10) est abaissée sur la boîte de croissance inférieure (10), et
la boîte comprend des moyens de manipulation de fluide configurés pour distribuer le fluide reçu des moyens de couplage de fluide de la partie supérieure (20) aux plantes ou aux insectes dans le plateau et pour collecter l'excès de fluide du plateau et le faire passer aux moyens de couplage de fluide de la partie inférieure (40).

2. Boîte (10) selon la revendication 1, dans laquelle la partie supérieure (20) et la partie inférieure (40) comprennent des moyens de couplage de puissance correspondants configurés pour fournir un couplage automatique de puissance électrique lorsqu'une boîte de croissance supérieure (10) est agencée sur le dessus d'une boîte de croissance inférieure (10).

3. Boîte (10) selon la revendication 1 ou 2, dans laquelle la partie supérieure (20) et la partie inférieure (40) comprennent des moyens de positionnement correspondants configurés pour fournir un alignement de position automatique lorsqu'une boîte de croissance supérieure (10) est abaissée sur le dessus d'une boîte de croissance inférieure (10).

4. Boîte (10) selon l'une quelconque des revendications précédentes, dans laquelle les moyens de couplage de fluide de la partie inférieure (40) comprennent une soupape qui ouvre le couplage de fluide lorsque la boîte est agencée sur le dessus d'une autre boîte inférieure (10).

5. Boîte (10) selon l'une quelconque des revendications précédentes, dans laquelle les moyens de couplage de fluide de la partie supérieure (20) comprennent une sortie de fluide (25) qui est couplée aux moyens de manipulation de fluide,
les deux entrées de fluide étant interconnectées de sorte que le fluide entrant au niveau de l'une quelconque des 2 entrées sort de la partie supérieure (20) au niveau de la sortie de fluide (25).

6. Boîte (10) selon l'une quelconque des revendications 2 à 5, dans laquelle les moyens de couplage de puissance fournissent une connexion électrique entre une colonne (31) de la boîte inférieure (10) et une colonne correspondante (31) de la boîte supérieure (10).

7. Boîte (10) selon la revendication 6, dans laquelle la puissance électrique est distribuée entre la partie supérieure et la partie inférieure via au moins une colonne (31).

8. Boîte (10) selon la revendication 6 ou 7, dans laquelle un dispositif de support (33) pour supporter la croissance des plantes ou des insectes dans la boîte est monté sur une barre (32) qui est fixée à au moins une colonne (31) fournissant de l'énergie électrique au dispositif de support (33).

9. Boîte (10) selon l'une quelconque des revendications précédentes, dans laquelle les moyens de manipulation de fluide comprennent une entrée de fluide (43) qui est couplée aux moyens de couplage de fluide de la partie supérieure (20), et des moyens de décharge de fluide agencés dans un cadre (41) de la partie inférieure (40) pour distribuer le fluide reçu aux plantes ou aux insectes dans le plateau.

10. Boîte (10) selon l'une quelconque des revendications précédentes, dans laquelle les moyens de manipulation de fluide comprennent un certain nombre de trous (45) agencés sur différents niveaux dans le cadre de la partie inférieure (40) pour recevoir l'excès de fluide du plateau et le faire passer aux moyens de couplage de fluide de la partie inférieure (40), les trous d'un même niveau étant connectés via un passage respectif (48), la boîte (10) comprenant en outre des moyens de commande de niveau de remplissage pour commander le niveau de remplissage de fluide du plateau en ouvrant ou fermant sélectivement le couplage entre le passage respectif (48) et les moyens de couplage de fluide de la partie inférieure (40).

11. Boîte (10) selon la revendication 10, dans laquelle les moyens de décharge de fluide sont agencés au niveau d'un côté du cadre (41) de la partie inférieure (40) et les trous (45) sont agencés au niveau du côté opposé du cadre (41) de la partie inférieure (40), les moyens de couplage de fluide de la partie inférieure (40) étant agencés au niveau d'une position qui est en dessous des moyens de couplage de fluide de la partie supérieure (40), et les passages (48) étant couplés via un canal (47) avec les moyens de couplage de fluide de la partie inférieure (40).

12. Boîte (10) selon l'une quelconque des revendications précédentes, comprenant en outre un couvercle (29) recouvrant la partie supérieure (20), dans laquelle un dispositif de support facultatif (27) pour supporter la croissance des plantes ou des insectes est monté sur le couvercle (29) et fourni avec une puissance électrique.

13. Système modulaire de culture de plantes ou d'élevage d'insectes (1) comprenant au moins une boîte empilée (10) selon l'une quelconque des revendications précédentes, et une station d'accueil (50) qui est configurée pour porter une première boîte de l'au moins une boîte empilée (10), la station d'accueil (50) comprenant des moyens de couplage de fluide configurés pour interagir avec les moyens de couplage de fluide de la partie inférieure (40) de la première boîte (10) pour fournir un couplage de fluide automatique lorsque la première boîte (10) est abaissée sur le dessus de la station d'accueil (50).

14. Système de culture ou d'élevage (1) selon la revendication 13, dans lequel la station d'accueil comprend des moyens de couplage de puissance configurés pour fournir un couplage automatique de puissance électrique avec la première boîte (10).
